# EUROPEAN PATENT APPLICATION

(11) **EP 4 071 180 A1**
(43) Date of publication of application: **12.10.2022**
(21) Application number: 20895428.9
(22) Date of filing: 09.01.2020
(51) Int. Cl.: C08B 37/02, C08B 37/00, C08B 37/08, C08B 31/00, A61K 31/785, A61P 29/00, A61P 31/00, A61P 31/04, A61P 17/02

(54) **POLYCATIONIC POLYSACCHARIDE AND APPLICATION THEREOF**

(30) Priority: 05.12.2019 CN 201911235541
(71) Applicant: Nanjing University, Nanjing, Jiangsu 210093 (CN)
(72) Inventor: DONG, Lei, Nanjing, Jiangsu 210000 (CN)
(74) Representative: JD&P Patent Attorneys Joanna Dargiewicz & Partners
(86) International application number: PCT/CN2020/071067
(87) International publication number: WO 2021/109317

(57) **Abstract**

A polycationic polysaccharide and an application thereof. Specifically, the polycationic polysaccharide consists of a polysaccharide and a polyamine compound, and is a positively charged polycationic polysaccharide obtained by reacting a polysaccharide with an amine-containing or polyamine compound. The polycationic polysaccharide is applied in a biomedical functional material of an antibacterial biofilm, a biomedical device, and an antibacterial functional material.

## Description

### FIELD

The present disclosure relates to the technical field of biomedicine, in particular to a polycationic polysaccharide and its applications as an antibacterial material and as a medicament for the treatment of chronic inflammatory disease.

### BACKGROUND

Natural polymer polysaccharides have good biocompatibility and bioactivity, and are widely used in clinical and biomedical fields. At present, semi-synthetic cationization-modified natural polysaccharides are most widely studied. They can be used as a carrier for delivery of a nucleic acid medicament, since the amino group on the surface modification can be densely positively charged after protonation in aqueous solution. At the same time, there is a plenty of literature reporting that the polysaccharide structure containing a large number of positive charges has excellent antibacterial properties. Cationized polysaccharides can also bind to biological macromolecules via charge interaction, affecting the related functions of the biological molecules, thereby changing the activity of the biological molecules.

Biofilm is a bacterial colony formed by bacteria, which is wrapped with bacterial extracellular macromolecules secreted by a variety of gram-negative bacteria or gram-positive bacteria, and thus adhered to the surface of an object. Biofilm can easily cause various infections. Biofilm coating can make cells protected by the extracellular macromolecules, thereby resisting against the body's immune defense, and can increase the resistance to antibiotics by 10-1000 times. The existence of biofilm greatly increases the difficulty in killing bacteria for traditional medicines, and promotes the occurrence of chronic infection or secondary infection. Therefore, there is a need to find a novel biofunctional material which can prevent biofilm formation and treat biofilm-related disorders, sterilize biomedical devices, and has better sterilization effects.

Currently, in clinical treatment, the formation and growth of biofilm is still a problem to be solved. The prevention and treatment of bacterial biofilm still remains at killing bacteria with antibiotics to reduce the formation of biofilm. However, the existence of biofilm will protect bacteria from the threat of antibiotics, leading to a vicious circle. Researchers found that polycationic polysaccharides are more effective than previous anti-bacterial biofilm agents because of their unique structure and positive charges, which results in excellent antibacterial activity and unique biological activity such as functions of promoting wound repair. Therefore, a polycationic polysaccharide can be used as a medicament applied in the treatment of infection caused by bacterial biofilm and related chronic inflammatory disease, and can be used for the development and application of bactericidal smears for biomedical devices and new antibacterial functional materials.

### SUMMARY

In view of the above-mentioned problems in the prior art, the present disclosure provides a polycationic polysaccharide, and the prepared polycationic polysaccharide can be used for the application in biomedical functional materials, biomedical devices and materials with antibacterial functions.

The technical solution of the present disclosure is as follows:
A polycationic polysaccharide, the polycationic polysaccharide is a positively charged polycationic polysaccharide obtained by the reaction between a polysaccharide and a polyamine compound, wherein the polysaccharide has the following general formula: 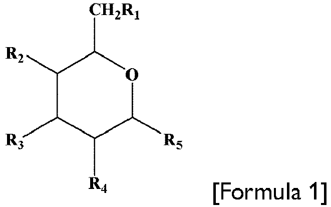 wherein
R1 to R5 are each independently selected from protected or unprotected hydroxyl group, protected or unprotected amino group, and sugar residue connected by glycosidic bond, and the sugar residue meets the requirements of Formula 1;
the polyamine compound has the following general formula: 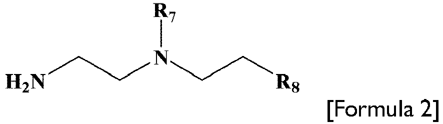 wherein
   R6 is selected from hydrogen atom or 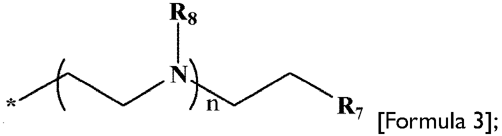
   R7 is selected from protected or unprotected amino group;
   R8 is selected from hydrogen atom or R6.

Preferably, the structural formula of the polycationic polysaccharide is: 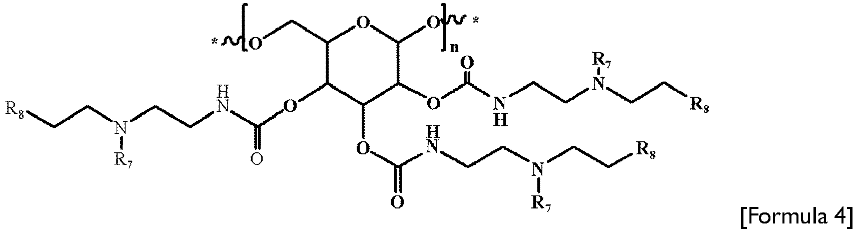 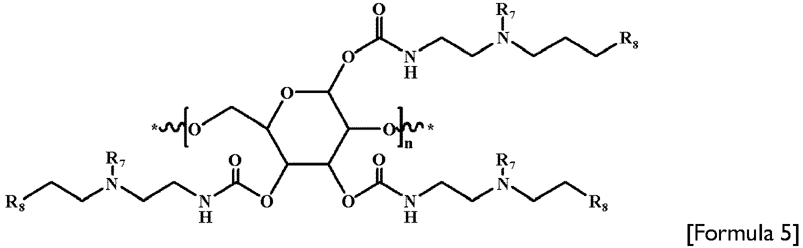 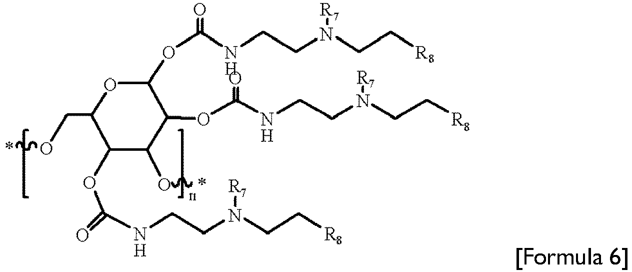 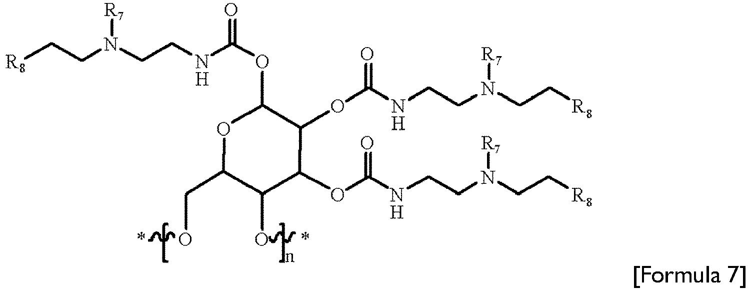
formula 4 is a polycationic polysaccharide composed of a polysaccharide molecule with (1→6) glycosidic bond as the main chain and grafted by a polyamine compound;
formula 5 is a polycationic polysaccharide composed of a polysaccharide molecule with (1→5) glycosidic bond as the main chain and grafted by a polyamine compound;
formula 6 is a polycationic polysaccharide composed of a polysaccharide molecule with (1→4) glycosidic bond as the main chain and grafted by a polyamine compound;
formula 7 is a polycationic polysaccharide composed of a polysaccharide molecule with (1→3) glycosidic bond as the main chain and grafted by a polyamine compound;
   wherein
   R6 is selected from hydrogen atom or 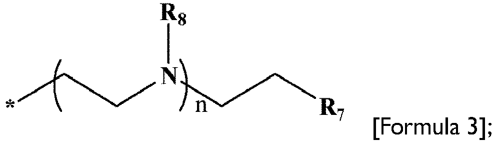
   R7 is selected from protected or unprotected amino group;
   R8 is selected from hydrogen atom or R6.

Preferably, the molecular weight of the polyamine compound is less than 500 Daltons, and the polyamine compound is any one of the following compounds:

| No | Compound | Structure |
|---|---|---|
| 1 | compound 1 | 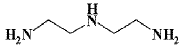 |
| 2 | compound 2 | 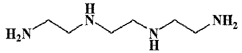 |
| 3 | compound 3 | 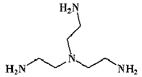 |
| 4 | compound 4 | 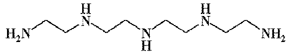 |
| 5 | compound 5 | 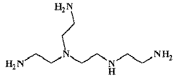 |
| 6 | compound 6 | 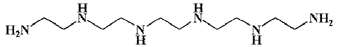 |
| 7 | compound 7 | 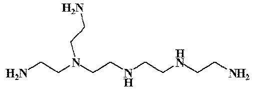 |
| 8 | compound 8 | 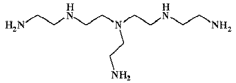 |
| 9 | compound 9 | 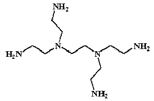 |
| 10 | compound 10 | 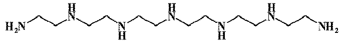 |
| 11 | compound 11 | 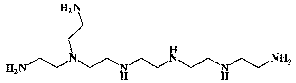 |
| 12 | compound 12 | 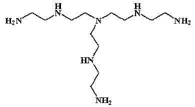 |
| 13 | compound 13 | 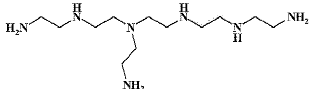 |
| 14 | compound 14 | 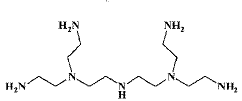 |
| 15 | compound 15 | 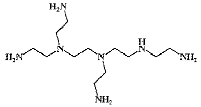 |
| 16 | compound 16 | 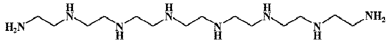 |
| 17 | compound 17 | 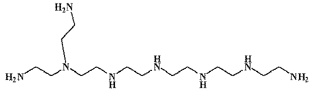 |
| 18 | compound 18 | 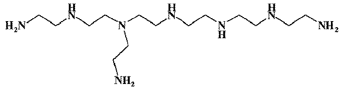 |
| 19 | compound 19 | 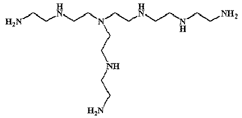 |
| 20 | compound 20 | 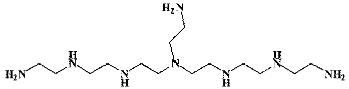 |
| 21 | compound 21 | 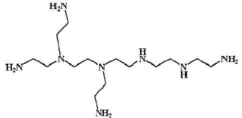 |
| 22 | compound 22 | 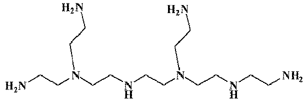 |
| 23 | compound 23 | 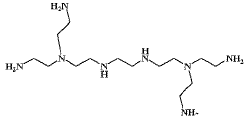 |
| 24 | compound 24 | 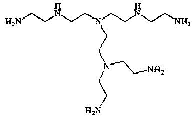 |
| 25 | compound 25 | 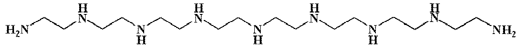 |
| 26 | compound 26 | 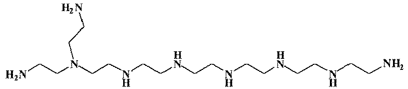 |
| 27 | compound 27 | 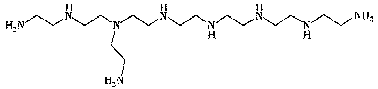 |
| 28 | compound 28 | 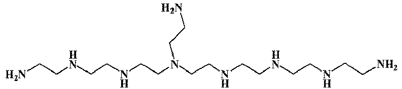 |
| 29 | compound 29 | 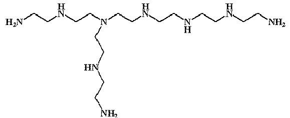 |
| 30 | compound 30 | 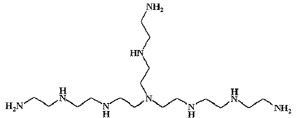 |
| 31 | compound 31 | 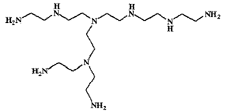 |
| 32 | compound 32 | 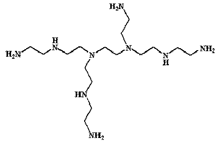 |
| 33 | compound 33 | 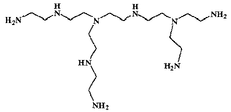 |
| 34 | compound 34 | 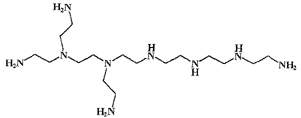 |
| 35 | compound 35 | 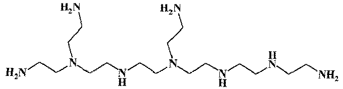 |
| 36 | compound 36 | 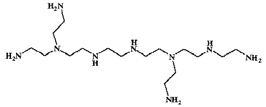 |
| 37 | compound 37 | 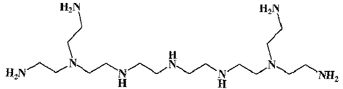 |
| 38 | compound 38 | 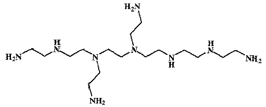 |
| 39 | compound 39 | 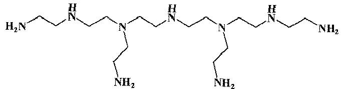 |
| 40 | compound 40 | 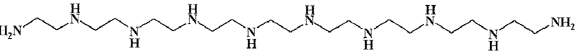 |
| 41 | compound 41 | 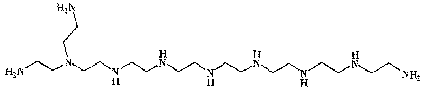 |
| 42 | compound 42 | 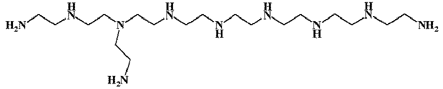 |
| 43 | compound 43 | 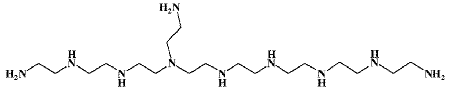 |
| 44 | compound 44 | 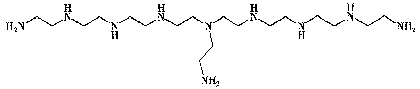 |
| 45 | compound 45 | 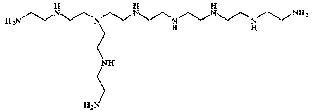 |
| 46 | compound 46 | 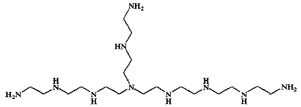 |
| 47 | compound 47 | 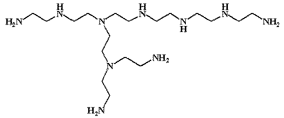 |
| 48 | compound 48 | 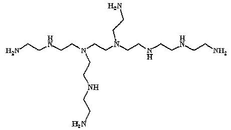 |
| 49 | compound 49 | 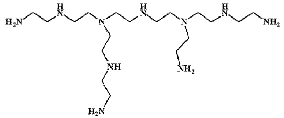 |
| 50 | compound 50 | 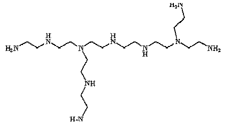 |
| 51 | compound 51 | 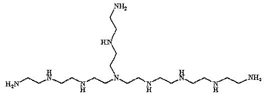 |
| 52 | compound 52 | 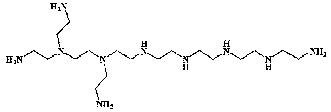 |
| 53 | compound 53 | 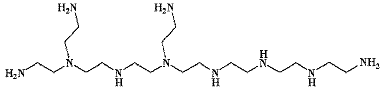 |
| 54 | compound 54 | 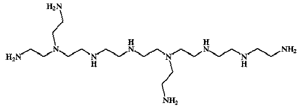 |
| 55 | compound 55 | 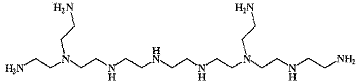 |
| 56 | compound 56 | 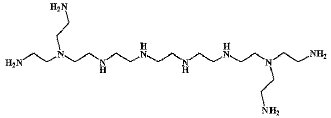 |
| 57 | compound 57 | 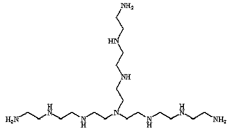 |
| 58 | compound 58 | 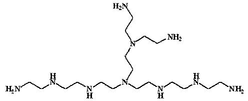 |
| 59 | compound 59 | 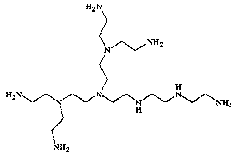 |
| 60 | compound 60 | 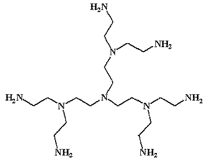 |
| 61 | compound 61 | 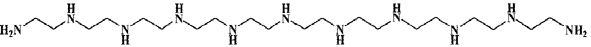 |
| 62 | compound 62 | 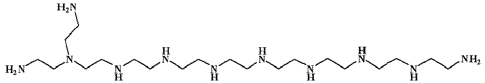 |
| 63 | compound 63 | 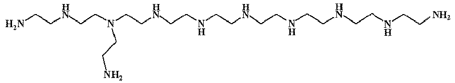 |
| 64 | compound 64 | 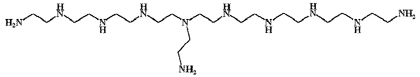 |
| 65 | compound 65 | 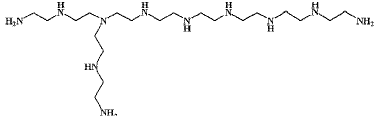 |
| 66 | compound 66 | 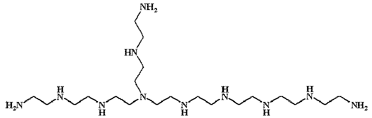 |
| 67 | compound 67 | 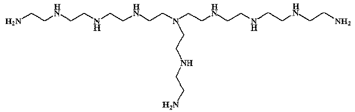 |
| 68 | compound 68 | 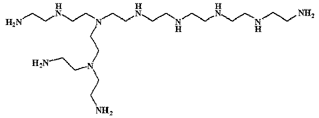 |
| 69 | compound 69 | 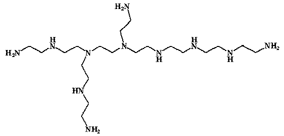 |
| 70 | compound 70 | 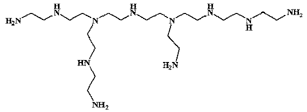 |
| 71 | compound 71 | 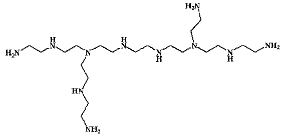 |
| 72 | compound 72 | 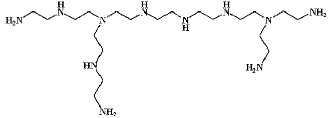 |
| 73 | compound 73 | 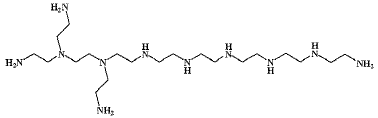 |
| 74 | compound 74 | 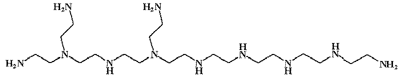 |
| 75 | compound 75 | 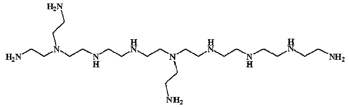 |
| 76 | compound 76 | 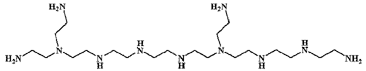 |
| 77 | compound 77 | 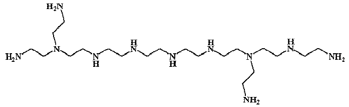 |
| 78 | compound 78 | 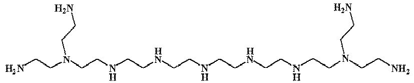 |
| 79 | compound 79 | 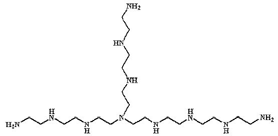 |
| 80 | compound 80 | 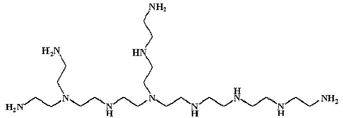 |
| 81 | compound 81 | 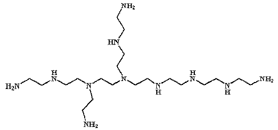 |
| 82 | compound 82 | 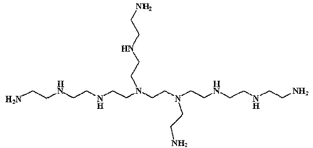 |
| 83 | compound 83 | 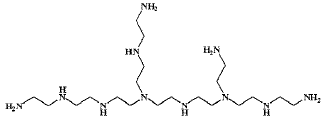 |
| 84 | compound 84 | 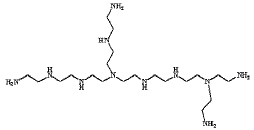 |
| 85 | compound 85 | 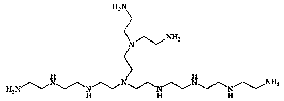 |
| 86 | compound 86 | 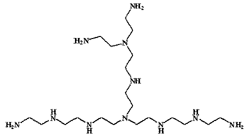 |
| 87 | compound 87 | 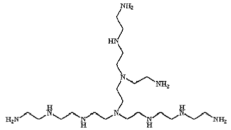 |
| 88 | compound 88 | 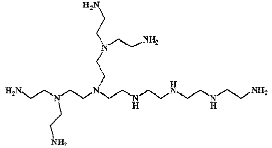 |
| 89 | compound 89 | 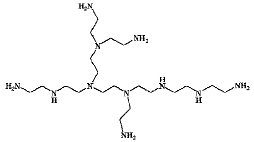 |
| 90 | compound 90 | 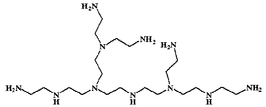 |
| 91 | compound 91 | 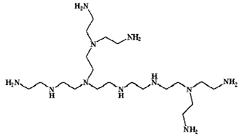 |
| 92 | compound 92 | 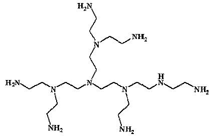 |
| 93 | compound 93 | 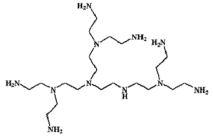 |
| 94 | compound 94 | 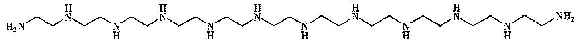 |
| 95 | compound 95 | 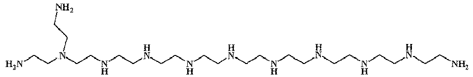 |
| 96 | compound 96 | 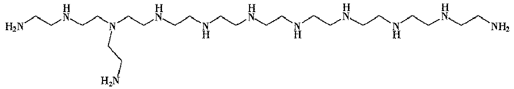 |
| 97 | compound 97 | 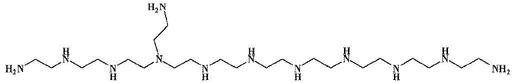 |
| 98 | compound 98 | 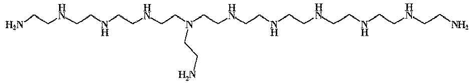 |
| 99 | compound 99 | 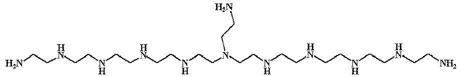 |
| 100 | compound 100 | 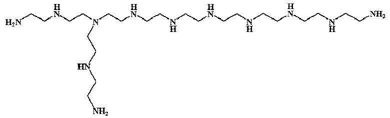 |
| 101 | compound 101 | 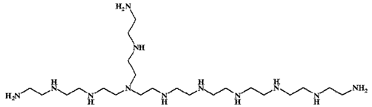 |
| 102 | compound 102 | 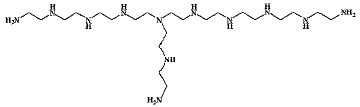 |
| 103 | compound 103 | 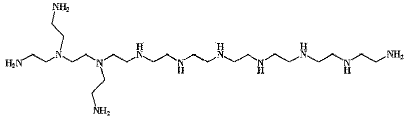 |
| 104 | compound 104 | 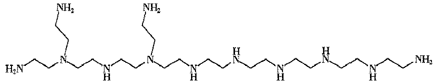 |
| 105 | compound 105 | 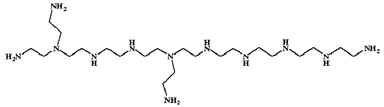 |
| 106 | compound 106 | 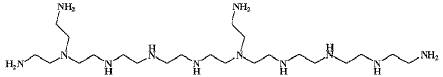 |
| 107 | compound 107 | 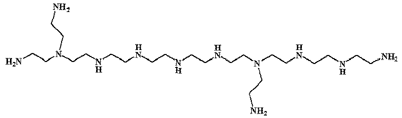 |
| 108 | compound 108 | 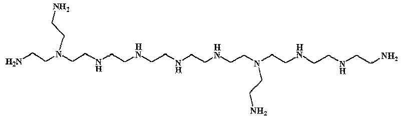 |
| 109 | compound 109 | 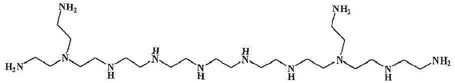 |
| 110 | compound 110 | 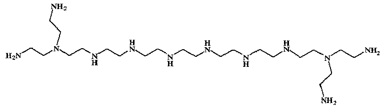 |
| 111 | compound 111 | 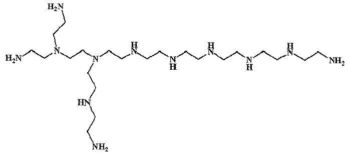 |
| 112 | compound 112 | 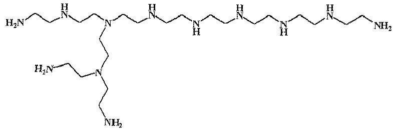 |
| 113 | compound 113 | 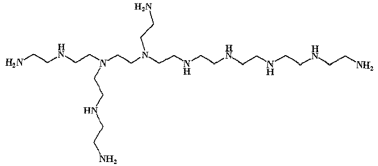 |
| 114 | compound 114 | 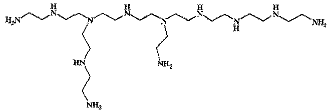 |
| 115 | compound 115 | 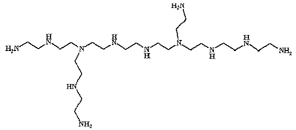 |
| 116 | compound 116 | 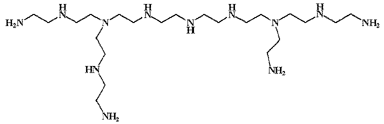 |
| 117 | compound 117 | 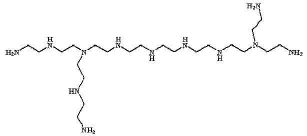 |
| 118 | compound 118 | 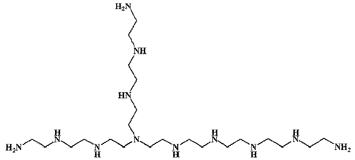 |
| 119 | compound 119 | 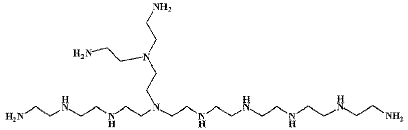 |
| 120 | compound 120 | 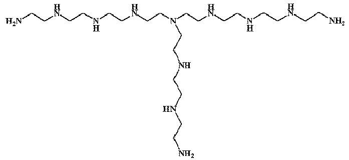 |
| 121 | compound 121 | 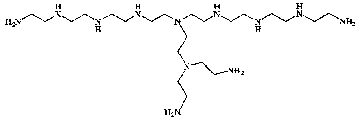 |
| 122 | compound 122 | 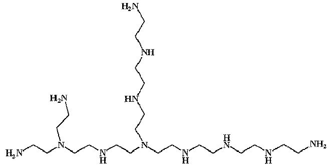 |
| 123 | compound 123 | 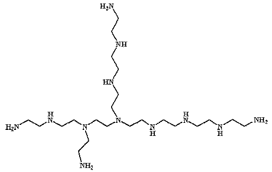 |
| 124 | compound 124 | 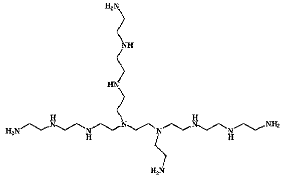 |
| 125 | compound 125 | 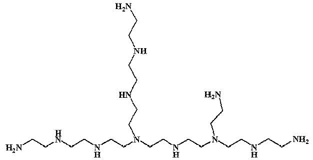 |
| 126 | compound 126 | 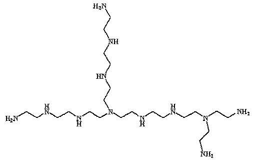 |
| 127 | compound 127 | 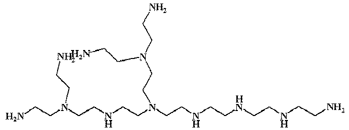 |
| 128 | compound 128 | 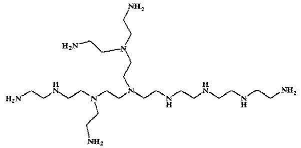 |
| 129 | compound 129 | 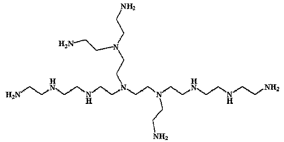 |
| 130 | compound 130 | 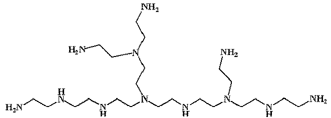 |
| 131 | compound 131 | 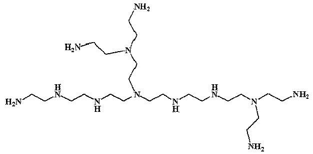 |
| 132 | compound 132 | 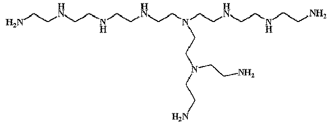 |
| 133 | compound 133 | 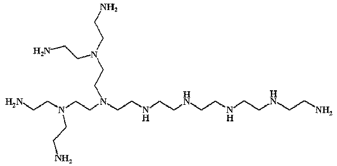 |
| 134 | compound 134 | 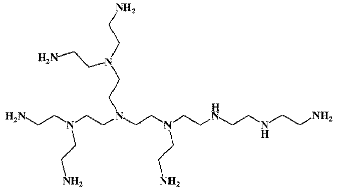 |
| 135 | compound 135 | 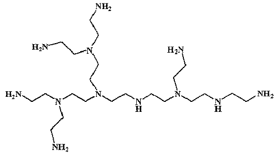 |
| 136 | compound 136 | 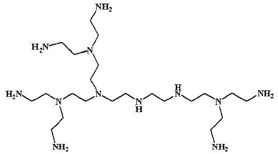 |
| 137 | compound 137 | 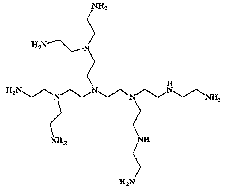 |
| 138 | compound 138 | 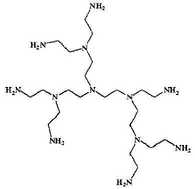 |
| 139 | compound 139 | 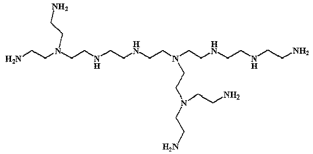 |
| 140 | compound 140 | 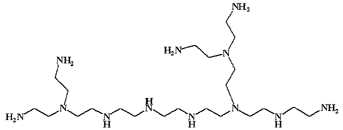 |
| 141 | compound 141 | 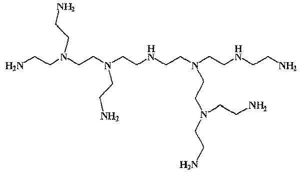 |
| 142 | compound 142 | 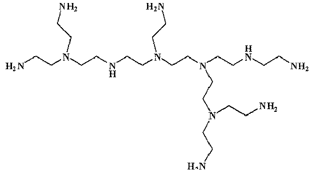 |

Preferably, the number of sugar units in the structure of the polysaccharide is 2 to 2000.

The present disclosure also discloses the use of the polycationic polysaccharide described above as an antibacterial material.

Preferably, the antibacterial material achieves the effect of killing bacteria by destroying the biofilm structures of the bacteria.

Preferably, the antibacterial material is used for the preparation of a medicament or a medical device for the prevention or treatment of Gram-negative and/or Gram-positive bacterial infection.

Preferably, the antibacterial material is used as a biomedical functional material or a biomedical device.

Preferably, the antibacterial material is an antibacterial functional material, including a daily chemical product, a packaging product, and a home improvement product with antibacterial functions.

The present disclosure also discloses an antibacterial agent, prepared from the polycationic polysaccharide described above as an active ingredient and a pharmaceutically acceptable adjuvant.

The present disclosure also discloses an antibacterial medical device, prepared from the polycationic polysaccharide described above as an active ingredient and a pharmaceutically acceptable adjuvant.

The present disclosure also discloses the use of the polycationic polysaccharide described above as a medicament for the treatment of chronic inflammatory disease.

Preferably, the medicament for the treatment of chronic inflammatory disease includes the medicament for the prevention of surgical wound infection, and the medicament for the prevention of scalding wound infection.

Compared with the prior art (such as the solution with application number of 201810714603.6), the polycationic polysaccharide of the present disclosure has better antibacterial and anti-inflammatory capacities, and functions of promoting wound healing, and has lower cytotoxicity, resulting in a great potential to be applied in biomedical devices and biomedical functional materials.

### BRIEF DESCRIPTION OF DRAWINGS

Figure 1 is the infrared spectrogram of the polycationic polysaccharide in example 1 of the present disclosure.
Figure 2 is the elemental analysis diagram of the polycationic polysaccharide in example 1 of the present disclosure.
Figure 3 is the H NMR spectrum of the polycationic polysaccharide in example 1 of the present disclosure.
Figure 4 is a graph showing the cytotoxicity results of the polycationic polysaccharide in example 1 of the present disclosure.
Figure 5 is a graph showing the tissue toxicity results of the polycationic polysaccharide in example 1 of the present disclosure.
Figure 6 is a diagram for the comparison of time in promoting wound healing for the polycationic polysaccharide of the present disclosure and the existing cationized polysaccharide.
Figure 7 is a diagram for the comparison of cytotoxicity for the polycationic polysaccharides of the present disclosure constructed with saccharides from different sources.
Figure 8 is a diagram for the comparison of time in promoting wound healing for the polycationic polysaccharides of the present disclosure constructed with saccharides from different sources.

### DETAILED DESCRIPTION

The following examples are further descriptions of the present disclosure to be an illustration of the present technical content, but the essential content of the present disclosure is not limited to the following examples. Those of ordinary skill in the art can and shall know that any simple changes or substitutions based on the essential spirit of the disclosure shall be within the protection scope of the present disclosure claimed.

### Example 1

A method for producing a polycationic polysaccharide, comprising the following steps:
1) Weighing 0.5 g of dextran with a molecular weight of 70,000 Daltons (purchased from Shanghai Macklin Biochemical Co., Ltd, cat # D806715), dissolving it in 25 ml dimethyl sulfoxide fully to obtain a mixed solution;
2) Weighing 1 g of N'N-carbonyldiimidazole, directly adding it to the dissolved dextran solution, and remaining reacting at room temperature for 2 hours;
3) Adding 2 g of diethylenetriamine dropwise to the solution obtained above, and remaining reacting at 25°C for 24 hours;
4) After the reaction completed, adding 5 times in volume of anhydrous ethanol to the solution obtained, stirring fully and then precipitating with centrifugation at 12,000 rpm for 10 minutes, then washing the obtained precipitate three times with anhydrous ethanol, and drying it in vacuum for 48 hours to yield a product, which was stored under dry conditions for later use, and named as DETA-Dex.

The specific reaction process is shown in the following formula:

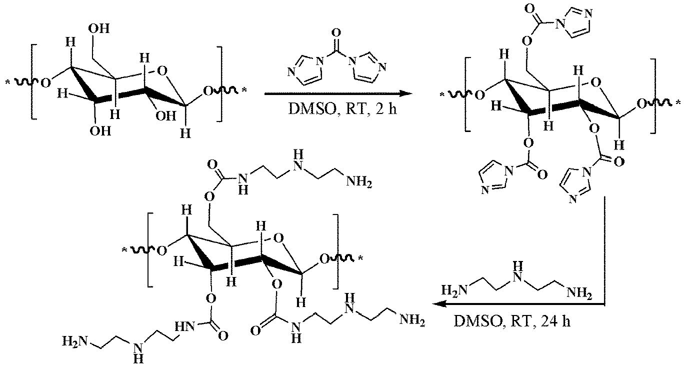

The prepared polycationic polysaccharide was characterized by infrared spectroscopy:
200 mg of potassium bromide and 2 mg of polycationic polysaccharide sample were weighted, and then ground in an agate mortar under baking with infrared lamp for the whole grinding process. The sample powder was placed into a mold and a pressure was applied up to 20 MPa. Maintained for 2 minutes, and then reduced the pressure to 0 slowly. The pressed sample tablet was took out and tested on a machine.

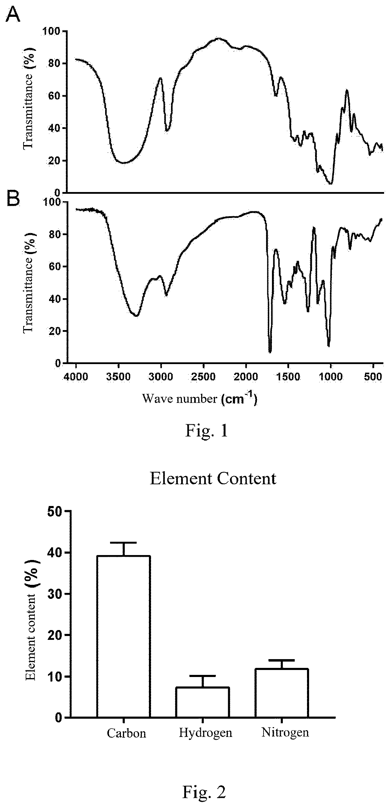

The results are shown in Figure 1. Figure A is the infrared spectrum of the dextran, and Figure B is the infrared spectrum of the polycationic polysaccharide. In Figure B, the peak at 1711 cm⁻¹ represents the stretching vibration peak of C=O in carbonyl, the peak at 1544 cm⁻¹ represents the bending vibration peak of the nitrogen-hydrogen bond in primary amino group, and the peak at 1022 cm⁻¹ represents the characteristic absorption peak of glucopyranose. The appearance of these peaks proved successful synthesis of the polycationic polysaccharide.

In addition, 5 mg of the prepared polycationic polysaccharide sample was weighted, then baked and ground fully under an infrared lamp. The sample powder was added to an elemental analyzer for testing. The results are shown in Figure 2, where the synthesis of the polycationic polysaccharide can be considered successful if the nitrogen content is more than 7%.

The prepared polycationic polysaccharide was characterized by H NMR spectrum:
5 mg of dextran sample and 5 mg of polycationic polysaccharide sample were weighted, and fully dissolved in 500 µl deuterated water respectively. Then the samples obtained were putted into a quartz NMR tube, and tested on a machine.

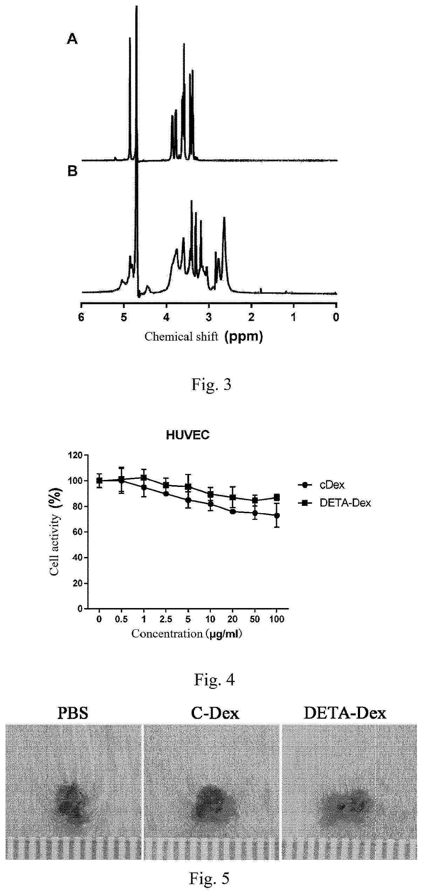

The results are shown in Figure 3. Figure A is the H NMR spectrum of the dextran, in which the peak at 3.34-3.97 ppm is the peak generated by the hydrogen in the sugar ring of the dextran molecule. Figure B is the H NMR spectrum of the polycationic polysaccharide, in which the peak at 2.94-4.00 ppm is the peak generated by the hydrogen in the sugar ring of the polycationic polysaccharide, and the peak at 2.49-2.87 ppm is the peak generated by the hydrogen to which the carbon atom in ethylene amino group (-CH2CH2NH) in diethylene triamine grafted on the sugar ring of the polycationic polysaccharide is connected. The appearance of this peak indicates that the polycationic polysaccharide was successfully synthesized.

### Example 2

Verification of cytotoxicity and tissue toxicity of the polycationic polysaccharide of the present disclosure.

### Cytotoxicity

Human umbilical vein epithelial cell HUVEC was selected, and inoculated into 96-well plate of cell culture at 10⁴ cells/well, and then pre-cultured for 24h. The cationized polysaccharide solution (cDex, derived from patent 201810714603.6) as prior art control group and the polycationic polysaccharide solution in this disclosure (named as DETA-Dex) were formulated with cell culture medium to a final concentration of 0.5 µg/ml, 1 µg/ml, 2.5 µg/ml, 5 µg/ml, 10 µg/ml, 20 µg/ml, 50 µg/ml, 100 µg/ml, respectively, and then added to the cell culture system for 30 min. After which, the cells were washed with cell culture medium for detection of cell activity.

Statistical results are shown in Figure 4, showing that for human umbilical vein epithelial cell HUVEC and human skin fibroblast HFF-1, the polycationic polysaccharide of the present disclosure has lower cytotoxicity, and has better biocompatibility.

### Tissue toxicity

### a. Establishment of a mouse back trauma model according to literature reports

Balb/c female mice were selected, weighed and recorded. The mice were randomized into groups with 10 mice per group. All the animals were intraperitoneally anesthetized with pentobarbital sodium. The back was dehaired and sterilized. At the thicker central part on the back of the mouse, a circular skin with a diameter of 0.5 cm was cut off to make a mouse back trauma model.

### b. Medicament treatment after modeling

In order to detect the tissue toxicity of the polycationic polysaccharide of the present disclosure to wound tissue, an experiment was performed as follows:
Blank control group: 100 µl of physiological saline was smeared to the wound area during administration;
Prior art control group: 100 µl of 1 mg/ml cationized polysaccharide solution (c-Dextran, hereinafter referred to as c-Dex, derived from patent 201810714603.6) was smeared to the wound area during administration;
Experimental group: 100 µl of 1 mg/ml polycationic polysaccharide solution (named DETA-Dex) was smeared to the wound area during administration;
The treated mice were placed in a warm, bright and comfortable environment to wait for them to wake up, and the wounds of the mice were examined 10 days later.

Statistical results are shown in Figure 5, showing that the mice smeared with the polycationic polysaccharide solution of the present disclosure exhibited an accelerated wound healing, and there was no obvious swelling and ulceration around the wound, which indicates that smearing the polycationic polysaccharide of the present disclosure can promote healing of wound and has no significant tissue toxicity.

### Example 3

Verification of therapeutic effect of the polycationic polysaccharide of the present disclosure on the model of wound infection by Pseudomonas aeruginosa.

### a. Establishment of a mouse back trauma model according to literature reports

Balb/c female mice were selected, weighed and recorded. The mice were randomized into groups with 10 mice per group. All the animals were intraperitoneally anesthetized with pentobarbital sodium. The back was dehaired and sterilized. At the thicker central part on the back of the mouse, a circular skin with a diameter of 0.5 cm was cut off to make a mouse back trauma model.

### b. Infection of mouse by Pseudomonas aeruginosa after modeling

Mice in each group were evenly smeared with Pseudomonas aeruginosa bacterial solution at the wound site at a dose of 10⁸ CFU/mouse, and the bacteria could form a complete biofilm within 72 hours.

### c. Medicament treatment

In order to detect the influence of the polycationic polysaccharide of the present disclosure on biofilm activity, an experiment was performed as follows:
Blank control group: 100 µl of physiological saline was smeared to the wound area during administration;
Prior art control group: 100 µl of 1 mg/ml cationized polysaccharide solution (cDex, derived from patent 201810714603.6) was smeared to the wound area during administration;
Experimental group: 100 µl of 1 mg/ml polycationic polysaccharide solution (named DETA-Dex) was smeared to the wound area during administration;

The treated mice were placed in a warm, bright and comfortable environment to wait for them to wake up. The wounds of the mice were detected every day. The time for complete wound healing was recorded, and the mean and standard deviation SD of the time for wound healing were calculated.

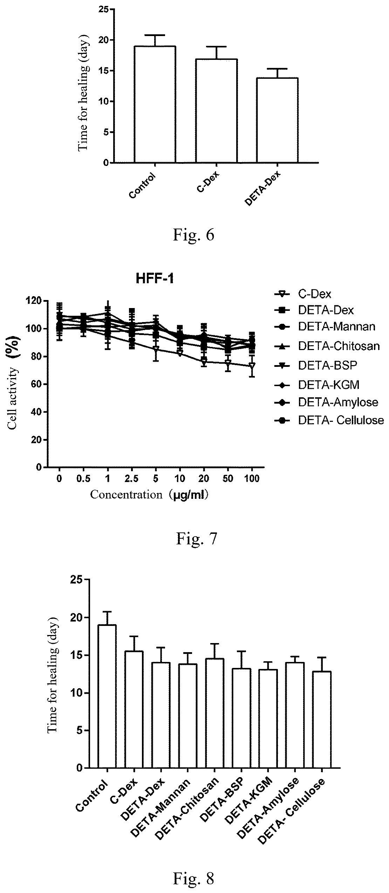

Statistical results are shown in Figure 6, showing that the mice smeared with the polycationic polysaccharide solution of the present disclosure exhibited an accelerated wound healing (the time for healing was the shortest), which indicates that smearing the polycationic polysaccharide of the present disclosure can quickly inhibit the proliferation and diffusion of bacteria and the formation of bacterial biofilm, effectively inhibit the production of endotoxin and exotoxin and the like by bacteria, and slow down development of disease.

### Example 4

A method for constructing the polycationic polysaccharide of the present disclosure with mannan, comprising the following steps:
1) Weighing 0.5 g of mannan with a molecular weight of 70,000 Daltons (purchased from Shanghai Macklin Biochemical Co., Ltd, cat # M861453), dissolving it in 25 ml dimethyl sulfoxide fully to obtain a mixed solution;
2) Weighing 1 g of N'N-carbonyldiimidazole, directly adding it to the dissolved mannan solution, and remaining reacting at room temperature for 2 hours;
3) Adding 2 g of diethylenetriamine dropwise to the solution obtained above, and remaining reacting at 25°C for 24 hours;
4) After the reaction completed, adding 5 times in volume of anhydrous ethanol to the solution obtained, stirring fully and then precipitating with centrifugation at 12,000 rpm for 10 minutes, then washing the obtained precipitate three times with anhydrous ethanol, and drying it in vacuum for 48 hours to yield a product, which was stored under dry conditions for later use, and named as DETA-Mannan.

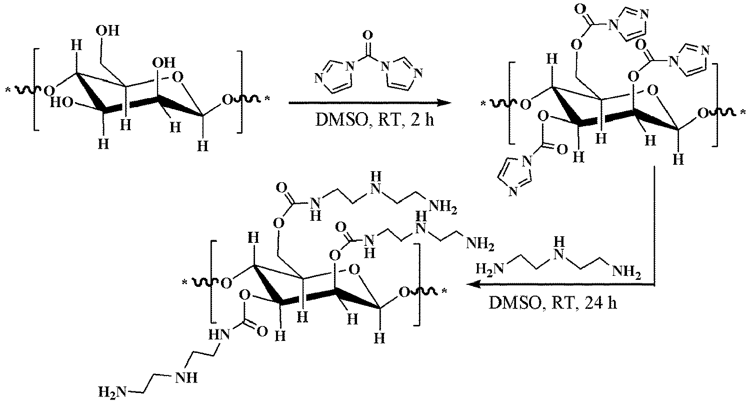

A method for constructing the polycationic polysaccharide of the present disclosure with chitosan, comprising the following steps:
1) Weighing 0.5 g of chitosan with a molecular weight of 70,000 Daltons (purchased from Shanghai Macklin Biochemical Co., Ltd, cat # C804726), dissolving it in 25 ml dimethyl sulfoxide fully to obtain a mixed solution;
2) Weighing 1 g of N'N-carbonyldiimidazole, directly adding it to the dissolved chitosan solution, and remaining reacting at room temperature for 2 hours;
3) Adding 2 g of diethylenetriamine dropwise to the solution obtained above, and remaining reacting at 25°C for 24 hours;
4) After the reaction completed, adding 5 times in volume of anhydrous ethanol to the solution obtained, stirring fully and then precipitating with centrifugation at 12,000 rpm for 10 minutes, then washing the obtained precipitate three times with anhydrous ethanol, and drying it in vacuum for 48 hours to yield a product, which was stored under dry conditions for later use, and named as DETA-chitosan.

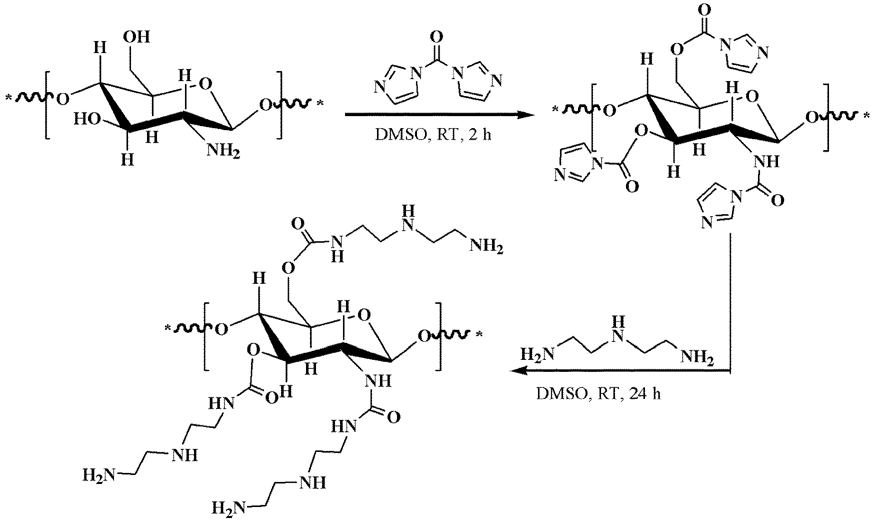

A method for constructing the polycationic polysaccharide of the present disclosure with Bletilla striata polysaccharide, comprising the following steps:
1) Weighing 0.5 g of Bletilla striata polysaccharide (purchased from Lanzhou wotelaisi Biotechnology Co., Ltd.), dissolving it in 25 ml dimethyl sulfoxide fully to obtain a mixed solution;
2) Weighing 1 g of N'N-carbonyldiimidazole, directly adding it to the dissolved Bletilla striata polysaccharide solution, and remaining reacting at room temperature for 2 hours;
3) Adding 2 g of diethylenetriamine dropwise to the solution obtained above, and remaining reacting at 25°C for 24 hours;
4) After the reaction completed, adding 5 times in volume of anhydrous ethanol to the solution obtained, stirring fully and then precipitating with centrifugation at 12,000 rpm for 10 minutes, then washing the obtained precipitate three times with anhydrous ethanol, and drying it in vacuum for 48 hours to yield a product, which was stored under dry conditions for later use, and named as DETA-BSP.

A method for constructing the polycationic polysaccharide of the present disclosure with konjac polysaccharide, comprising the following steps:
1) Weighing 0.5 g of konjac polysaccharide (purchased from Lanzhou wotelaisi Biotechnology Co., Ltd.), dissolving it in 25 ml dimethyl sulfoxide fully to obtain a mixed solution;
2) Weighing 1 g of N'N-carbonyldiimidazole, directly adding it to the dissolved konjac polysaccharide solution, and remaining reacting at room temperature for 2 hours;
3) Adding 2 ml of diethylenetriamine dropwise to the solution obtained above, and remaining reacting at 25°C for 24 hours;
4) After the reaction completed, adding 5 times in volume of anhydrous ethanol to the solution obtained, stirring fully and then precipitating with centrifugation at 12,000 rpm for 10 minutes, then washing the obtained precipitate three times with anhydrous ethanol, and drying it in vacuum for 48 hours to yield a product, which was stored under dry conditions for later use, and named as DETA-KGM.

A method for constructing the polycationic polysaccharide of the present disclosure with amylose, comprising the following steps:
1) Weighing 0.5 g of amylose (Shanghai Macklin Biochemical Co., Ltd, cat # S817547), dissolving it in 25 ml dimethyl sulfoxide fully to obtain a mixed solution;
2) Weighing 1 g of N'N-carbonyldiimidazole, directly adding it to the dissolved amylose solution, and remaining reacting at room temperature for 2 hours;
3) Adding 2 g of diethylenetriamine dropwise to the solution obtained above, and remaining reacting at 25°C for 24 hours;
4) After the reaction completed, adding 5 times in volume of anhydrous ethanol to the solution obtained, stirring fully and then precipitating with centrifugation at 12,000 rpm for 10 minutes, then washing the obtained precipitate three times with anhydrous ethanol, and drying it in vacuum for 48 hours to yield a product, which was stored under dry conditions for later use, and named as DETA-amylose.

A method for constructing the polycationic polysaccharide of the present disclosure with cellulose, comprising the following steps:
1) Weighing 0.5 g of cellulose (Shanghai Macklin Biochemical Co., Ltd, 25µm, cat # C804602), dissolving it in 25 ml dimethyl sulfoxide fully to obtain a mixed solution;
2) Weighing 1 g of N'N-carbonyldiimidazole, directly adding it to the dissolved cellulose solution, and remaining reacting at room temperature for 2 hours;
3) Adding 2 g of diethylenetriamine dropwise to the solution obtained above, and remaining reacting at 25°C for 24 hours;
4) After the reaction completed, adding 5 times in volume of anhydrous ethanol to the solution obtained, stirring fully and then precipitating with centrifugation at 12,000 rpm for 10 minutes, then washing the obtained precipitate three times with anhydrous ethanol, and drying it in vacuum for 48 hours to yield a product, which was stored under dry conditions for later use, and named as DETA-cellulose.

A method for constructing the polycationic polysaccharide of the present disclosure with different polyamine compounds, comprising the following steps:
1) Weighing 0.5 g of dextran with a molecular weight of 70,000 Daltons, dissolving it in 25 ml dimethyl sulfoxide fully to obtain a mixed solution;
2) Weighing 1 g of N'N-carbonyldiimidazole, directly adding it to the dissolved dextran solution, and remaining reacting at room temperature for 2 hours;
3) Adding 2 g of compound 2 to compound 142 respectively to the solution obtained above, and remaining reacting at 25°C for 24 hours;
4) After the reaction completed, adding 5 times in volume of anhydrous ethanol to the solution obtained, stirring fully and then precipitating with centrifugation at 12,000 rpm for 10 minutes, then washing the obtained precipitates three times with anhydrous ethanol, and drying them in vacuum for 48 hours to yield products, which were stored under dry conditions for later use, and named as 2-Dex to142-Dex respectively.

A method for constructing the polycationic polysaccharide of the present disclosure with dextran of different molecular weights, comprising the following steps:
1) Weighing 0.5 g of dextran with molecular weights of 360 Daltons, 50,000 Daltons, and 304,000 Daltons, respectively, dissolving each of them in 25 ml dimethyl sulfoxide fully to obtain a mixed solution;
2) For each dissolved dextran solution, weighing 1 g of N'N-carbonyldiimidazole, directly adding it to the dextran solution, and remaining reacting at room temperature for 2 hours;
3) Adding 2 g of diethylenetriamine to the solution obtained above respectively, and remaining reacting at 25°C for 24 hours;
4) After the reaction completed, adding 5 times in volume of anhydrous ethanol to the solution obtained, stirring fully and then precipitating with centrifugation at 12,000 rpm for 10 minutes, then washing the obtained precipitates three times with anhydrous ethanol, and drying them in vacuum for 48 hours to yield products, which were stored under dry conditions for later use, and named as DETA-0.36, DETA-50, DETA-304 respectively.

A method for constructing the polycationic polysaccharide of the present disclosure with different polyamine compounds and mannan, comprising the following steps:
1) Weighing 0.5 g of dextran with a molecular weight of 70,000 Daltons, dissolving it in 25 ml dimethyl sulfoxide fully to obtain a mixed solution;
2) Weighing 1 g of N'N-carbonyldiimidazole, directly adding it to the dissolved dextran solution, and remaining reacting at room temperature for 2 hours;
3) Adding 2 g of compound 7, compound 15, compound 92 and compound 128 respectively to the solution obtained above, and remaining reacting at 25°C for 24 hours;
4) After the reaction completed, adding 5 times in volume of anhydrous ethanol to the solution obtained, stirring fully and then precipitating with centrifugation at 12,000 rpm for 10 minutes, then washing the obtained precipitates three times with anhydrous ethanol, and drying them in vacuum for 48 hours to yield products, which were stored under dry conditions for later use, and named as 7-Mannan, 15-Mannan, 92-Mannan, and 128-Mannan respectively.

A method for constructing the polycationic polysaccharide of the present disclosure with different polyamine compounds and chitosan, comprising the following steps:
1) Weighing 0.5 g of chitosan with a molecular weight of 70,000 Daltons, dissolving it in 25 ml dimethyl sulfoxide fully to obtain a mixed solution;
2) Weighing 1 g of N'N-carbonyldiimidazole, directly adding it to the dissolved chitosan solution, and remaining reacting at room temperature for 2 hours;
3) Adding 2 g of compound 2, compound 61 and compound 94 respectively to the solution obtained above, and remaining reacting at 25°C for 24 hours;
4) After the reaction completed, adding 5 times in volume of anhydrous ethanol to the solution obtained, stirring fully and then precipitating with centrifugation at 12,000 rpm for 10 minutes, then washing the obtained precipitates three times with anhydrous ethanol, and drying them in vacuum for 48 hours to yield products, which were stored under dry conditions for later use, and named as 2-chitosan, 61-chitosan, and 94-chitosan respectively.

A method for constructing the polycationic polysaccharide of the present disclosure with different polyamine compounds and Bletilla striata polysaccharide, comprising the following steps:
1) Weighing 0.5 g of Bletilla striata polysaccharide with a molecular weight of 70,000 Daltons, dissolving it in 25 ml dimethyl sulfoxide fully to obtain a mixed solution;
2) Weighing 1 g of N'N-carbonyldiimidazole, directly adding it to the dissolved Bletilla striata polysaccharide solution, and remaining reacting at room temperature for 2 hours;
3) Adding 2 g of compound 4, compound 14, compound 62 and compound 103 respectively to the solution obtained above, and remaining reacting at 25°C for 24 hours;
4) After the reaction completed, adding 5 times in volume of anhydrous ethanol to the solution obtained, stirring fully and then precipitating with centrifugation at 12,000 rpm for 10 minutes, then washing the obtained precipitates three times with anhydrous ethanol, and drying them in vacuum for 48 hours to yield products, which were stored under dry conditions for later use, and named as 4-BSP, 14-BSP, 62-BSP, and 103-BSP respectively.

A method for constructing the polycationic polysaccharide of the present disclosure with different polyamine compounds and konjac polysaccharide, comprising the following steps:
1) Weighing 0.5 g of konjac polysaccharide with a molecular weight of 70,000 Daltons, dissolving it in 25 ml dimethyl sulfoxide fully to obtain a mixed solution;
2) Weighing 1 g of N'N-carbonyldiimidazole, directly adding it to the dissolved konjac polysaccharide solution, and remaining reacting at room temperature for 2 hours;
3) Adding 2 g of compound 8, compound 44, compound 67 and compound 102 respectively to the solution obtained above, and remaining reacting at 25°C for 24 hours;
4) After the reaction completed, adding 5 times in volume of anhydrous ethanol to the solution obtained, stirring fully and then precipitating with centrifugation at 12,000 rpm for 10 minutes, then washing the obtained precipitates three times with anhydrous ethanol, and drying them in vacuum for 48 hours to yield products, which were stored under dry conditions for later use, and named as 8-KGM, 44-KGM, 67-KGM, and 102-KGM respectively.

A method for constructing the polycationic polysaccharide of the present disclosure with different polyamine compounds and amylose, comprising the following steps:
1) Weighing 0.5 g of amylose with a molecular weight of 70,000 Daltons, dissolving it in 25 ml dimethyl sulfoxide fully to obtain a mixed solution;
2) Weighing 1 g of N'N-carbonyldiimidazole, directly adding it to the dissolved amylose solution, and remaining reacting at room temperature for 2 hours;
3) Adding 2 g of compound 15, compound 37 and compound 112 respectively to the solution obtained above, and remaining reacting at 25°C for 24 hours;
4) After the reaction completed, adding 5 times in volume of anhydrous ethanol to the solution obtained, stirring fully and then precipitating with centrifugation at 12,000 rpm for 10 minutes, then washing the obtained precipitates three times with anhydrous ethanol, and drying them in vacuum for 48 hours to yield products, which were stored under dry conditions for later use, and named as 15-Amylose, 37-Amylose and 111-Amylose respectively.

A method for constructing the polycationic polysaccharide of the present disclosure with different polyamine compounds and cellulose, comprising the following steps:
1) Weighing 0.5 g of cellulose with a molecular weight of 70,000 Daltons, dissolving it in 25 ml dimethyl sulfoxide fully to obtain a mixed solution;
2) Weighing 1 g of N'N-carbonyldiimidazole, directly adding it to the dissolved cellulose solution, and remaining reacting at room temperature for 2 hours;
3) Adding 2 g of compound 10, compound 49, compound 87, compound 102 and compound 140 respectively to the solution obtained above, and remaining reacting at 25°C for 24 hours;
4) After the reaction completed, adding 5 times in volume of anhydrous ethanol to the solution obtained, stirring fully and then precipitating with centrifugation at 12,000 rpm for 10 minutes, then washing the obtained precipitates three times with anhydrous ethanol, and drying them in vacuum for 48 hours to yield products, which were stored under dry conditions for later use, and named as 10-Cellulose, 49-Cellulose, 87-Cellulose, 102-Cellulose, and 140-Cellulose respectively.

### Example 5

Verification of cytotoxicity of the polycationic polysaccharide of the present disclosure.

Human umbilical vein epithelial cell HUVEC was selected, and inoculated into 96-well plate of cell culture at 10⁴ cells/well, and then pre-cultured for 24h. The polycationic polysaccharide solution in this disclosure (named as DETA-Dex, DETA-Mannan, DETA-Chitosan, DETA-BSP, DETA-KGM, DETA-Amylose, DETA-Cellulose) were formulated with cell culture medium to a final concentration of 0.5 µg/ml, 1 µg/ml, 2.5 µg/ml, 5 µg/ml, 10 µg/ml, 20 µg/ml, 50 µg/ml, 100 µg/ml, respectively, and then added to the cell culture system for 30 min. After which, the cells were washed with cell culture medium for detection of cell activity.

Statistical results are shown in Figure 7, showing that for human umbilical vein epithelial cell HUVEC, the polycationic polysaccharide of the present disclosure has lower cytotoxicity, and has better biocompatibility. For actual application, higher concentrations and larger doses can achieve better therapeutic effects.

### Example 6

Verification of therapeutic effect of the polycationic polysaccharides of the present disclosure constructed with different polysaccharides on the model of wound infection by Pseudomonas aeruginosa.

### a. Establishment of a mouse back trauma model according to literature reports

Balb/c female mice were selected, weighed and recorded. The mice were randomized into groups with 10 mice per group. All the animals were intraperitoneally anesthetized with pentobarbital sodium. The back was dehaired and sterilized. At the thicker central part on the back of the mouse, a circular skin with a diameter of 0.5 cm was cut off to make a mouse back trauma model.

### b. Infection of mouse by Pseudomonas aeruginosa after modeling

Mice in each group were evenly smeared with Pseudomonas aeruginosa bacterial solution at the wound site at a dose of 10⁸ CFU/mouse, and the bacteria could form a complete biofilm within 72 hours.

### c. Medicament treatment

In order to detect the influence of the polycationic polysaccharides of the present disclosure on biofilm activity, an experiment was performed as follows:
Blank control group: physiological saline was smeared to the wound area during administration;
Experimental group: 100 µl of 1 mg/ml polycationic polysaccharide solution (named as DETA-Dex, DETA-Mannan, DETA-Chitosan, DETA-BSP, DETA-KGM, DETA-Amylose, DETA-Cellulose, 2-Dex to 142-Dex, DETA-0.36, DETA-50, DETA-304, 7-Mannan, 15-Mannan, 92-Mannan, 128-Mannan, 2-chitosan, 61-chitosan, 94-chitosan, 4-BSP, 14-BSP, 62-BSP, 103-BSP, 8-KGM, 44-KGM, 67-KGM, 102-KGM, 15-Amylose, 37-Amylose, 111-Amylose, 10-Cellulose, 49-Cellulose, 87-Cellulose, 102-Cellulose, 140-Cellulose) was smeared to the wound area during administration;

The treated mice were placed in a warm, bright and comfortable environment to wait for them to wake up. The wounds of the mice were detected every day. The time for complete wound healing was recorded, and the mean and standard deviation SD of the time for wound healing were calculated.

Statistical results are shown in Figure 8 and Table 1, showing that all the mice smeared with the polycationic polysaccharide solution of the present disclosure constructed with polysaccharides from different sources exhibited an accelerated wound healing, which indicates that smearing the polycationic polysaccharide of the present disclosure can quickly inhibit the proliferation and diffusion of bacteria and the formation of bacterial biofilm, effectively inhibit the production of endotoxin and exotoxin and the like by bacteria, and slow down development of disease.

**Table 1. Time for wound healing of mice treated with polycationic polysaccharide**

| Name of polycationic polysaccharide | Time for wound healing (day) | Standard deviation |
|---|---|---|
| 2-Dex | 15.64 | 1.42 |
| 3-Dex | 15.21 | 1.18 |
| 4-Dex | 13.97 | 0.95 |
| 5-Dex | 15.80 | 0.92 |
| 6-Dex | 13.84 | 0.54 |
| 7-Dex | 15.58 | 0.51 |
| 8-Dex | 14.96 | 0.87 |
| 9-Dex | 15.24 | 1.08 |
| 10-Dex | 13.84 | 0.87 |
| 11-Dex | 14.35 | 0.58 |
| 12-Dex | 15.22 | 1.05 |
| 13-Dex | 14.50 | 0.98 |
| 14-Dex | 14.88 | 1.26 |
| 15-Dex | 15.22 | 1.15 |
| 16-Dex | 14.83 | 0.78 |
| 17-Dex | 15.99 | 1.47 |
| 18-Dex | 15.91 | 1.25 |
| 19-Dex | 14.78 | 1.31 |
| 20-Dex | 13.66 | 0.91 |
| 21-Dex | 14.66 | 1.04 |
| 22-Dex | 14.21 | 0.78 |
| 23-Dex | 14.23 | 0.96 |
| 24-Dex | 14.24 | 1.20 |
| 25-Dex | 14.44 | 1.39 |
| 26-Dex | 16.01 | 1.30 |
| 27-Dex | 14.75 | 1.19 |
| 28-Dex | 15.87 | 1.14 |
| 29-Dex | 13.58 | 0.88 |
| 30-Dex | 14.11 | 1.47 |
| 31-Dex | 15.71 | 0.82 |
| 32-Dex | 15.78 | 1.11 |
| 33-Dex | 14.81 | 1.03 |
| 34-Dex | 15.67 | 1.31 |
| 35-Dex | 14.70 | 0.66 |
| 36-Dex | 14.81 | 0.53 |
| 37-Dex | 13.73 | 0.83 |
| 38-Dex | 13.58 | 0.78 |
| 39-Dex | 15.55 | 0.78 |
| 40-Dex | 14.09 | 1.39 |
| 41-Dex | 13.60 | 0.59 |
| 42-Dex | 14.88 | 0.68 |
| 43-Dex | 14.47 | 0.87 |
| 44-Dex | 14.14 | 0.97 |
| 45-Dex | 14.89 | 0.68 |
| 46-Dex | 14.19 | 1.32 |
| 47-Dex | 14.12 | 0.93 |
| 48-Dex | 15.95 | 1.42 |
| 49-Dex | 13.73 | 1.37 |
| 50-Dex | 15.58 | 1.13 |
| 51-Dex | 14.00 | 0.51 |
| 52-Dex | 15.78 | 1.39 |
| 53-Dex | 14.57 | 0.95 |
| 54-Dex | 15.48 | 0.93 |
| 55-Dex | 15.53 | 0.99 |
| 56-Dex | 14.84 | 1.14 |
| 57-Dex | 14.29 | 0.64 |
| 58-Dex | 13.61 | 0.92 |
| 59-Dex | 15.62 | 1.48 |
| 60-Dex | 13.87 | 1.28 |
| 61-Dex | 15.71 | 0.84 |
| 62-Dex | 14.69 | 0.57 |
| 63-Dex | 15.68 | 1.48 |
| 64-Dex | 15.65 | 0.99 |
| 65-Dex | 14.52 | 0.74 |
| 66-Dex | 15.98 | 0.85 |
| 67-Dex | 15.65 | 1.18 |
| 68-Dex | 15.90 | 1.33 |
| 69-Dex | 15.41 | 0.85 |
| 70-Dex | 13.81 | 0.66 |
| 71-Dex | 14.48 | 1.49 |
| 72-Dex | 15.34 | 1.13 |
| 73-Dex | 13.95 | 1.10 |
| 74-Dex | 15.66 | 1.47 |
| 75-Dex | 13.61 | 0.54 |
| 76-Dex | 13.76 | 1.31 |
| 77-Dex | 14.12 | 1.32 |
| 78-Dex | 15.39 | 0.76 |
| 79-Dex | 15.61 | 0.62 |
| 80-Dex | 15.96 | 0.98 |
| 81-Dex | 15.10 | 0.73 |
| 82-Dex | 15.06 | 1.21 |
| 83-Dex | 14.12 | 1.32 |
| 84-Dex | 15.86 | 1.04 |
| 85-Dex | 13.66 | 0.89 |
| 86-Dex | 15.92 | 1.45 |
| 87-Dex | 15.85 | 1.13 |
| 88-Dex | 14.13 | 0.81 |
| 89-Dex | 15.07 | 1.25 |
| 90-Dex | 14.21 | 1.23 |
| 91-Dex | 15.18 | 0.77 |
| 92-Dex | 14.61 | 0.60 |
| 93-Dex | 15.27 | 1.14 |
| 94-Dex | 14.16 | 1.43 |
| 95-Dex | 15.38 | 1.26 |
| 96-Dex | 14.39 | 0.82 |
| 97-Dex | 16.01 | 1.25 |
| 98-Dex | 14.28 | 0.59 |
| 99-Dex | 14.87 | 1.22 |
| 100-Dex | 13.83 | 0.71 |
| 101-Dex | 15.10 | 0.65 |
| 102-Dex | 15.08 | 1.10 |
| 103-Dex | 15.22 | 1.27 |
| 104-Dex | 14.22 | 1.02 |
| 105-Dex | 14.49 | 1.36 |
| 106-Dex | 14.63 | 0.74 |
| 107-Dex | 15.36 | 1.28 |
| 108-Dex | 14.17 | 0.79 |
| 109-Dex | 15.30 | 1.04 |
| 110-Dex | 15.20 | 0.86 |
| 111-Dex | 15.13 | 1.25 |
| 112-Dex | 14.65 | 0.76 |
| 113-Dex | 14.77 | 0.86 |
| 114-Dex | 15.66 | 0.57 |
| 115-Dex | 14.51 | 1.40 |
| 116-Dex | 15.81 | 1.45 |
| 117-Dex | 13.91 | 1.19 |
| 118-Dex | 15.34 | 1.43 |
| 119-Dex | 14.34 | 1.39 |
| 120-Dex | 14.63 | 0.68 |
| 121-Dex | 15.66 | 1.42 |
| 122-Dex | 14.21 | 0.90 |
| 123-Dex | 13.69 | 1.16 |
| 124-Dex | 15.14 | 1.35 |
| 125-Dex | 15.25 | 1.23 |
| 126-Dex | 13.58 | 0.67 |
| 127-Dex | 14.10 | 0.54 |
| 128-Dex | 15.09 | 1.30 |
| 129-Dex | 14.08 | 0.58 |
| 130-Dex | 15.90 | 0.91 |
| 131-Dex | 14.77 | 1.17 |
| 132-Dex | 14.97 | 1.47 |
| 133-Dex | 15.58 | 1.48 |
| 134-Dex | 14.39 | 1.28 |
| 135-Dex | 15.96 | 0.95 |
| 136-Dex | 14.01 | 1.31 |
| 137-Dex | 15.26 | 0.73 |
| 138-Dex | 15.35 | 1.19 |
| 139-Dex | 14.99 | 0.71 |
| 140-Dex | 14.16 | 1.20 |
| 141-Dex | 13.93 | 0.91 |
| 142-Dex | 15.69 | 1.38 |
| DETA-0.36 | 17.38 | 2.41 |
| DETA-50 | 13.2 | 2.67 |
| DETA-304 | 16.31 | 1.38 |
| 7-Mannan | 15.77 | 2.11 |
| 15-Mannan | 15.54 | 1.71 |
| 92-Mannan | 14.84 | 1.00 |
| 128-Mannan | 14.99 | 1.33 |
| 2-chitosan | 15.14 | 0.55 |
| 61-chitosan | 14.95 | 1.51 |
| 94-chitosan | 15.52 | 1.14 |
| 4-BSP | 15.92 | 0.33 |
| 14-BSP | 14.75 | 1.27 |
| 62-BSP | 14.09 | 1.46 |
| 103-BSP | 14.45 | 2.35 |
| 8-KGM | 13.98 | 1.31 |
| 44-KGM | 15.16 | 1.28 |
| 67-KGM | 14.86 | 2.34 |
| 102-KGM | 14.01 | 1.76 |
| 15-Amylose | 15.12 | 0.27 |
| 37-Amylose | 14.04 | 1.71 |
| 111-Amylose | 14.99 | 1.86 |
| 10-Cellulose | 14.41 | 1.99 |
| 49-Cellulose | 14.49 | 1.25 |
| 87-Cellulose | 15.36 | 0.53 |
| 102-Cellulose | 15.45 | 1.77 |
| 140-Cellulose | 15.19 | 0.76 |

The above descriptions are only preferred examples of the present disclosure and are not intended to limit the present disclosure. Any modification, equivalent replacement or improvement made within the spirit and principle of the present disclosure shall be included in the protection scope of the present disclosure.

## Claims

1. A polycationic polysaccharide, the polycationic polysaccharide is a positively charged polycationic polysaccharide obtained by the reaction between a polysaccharide and a polyamine compound, wherein the polysaccharide has the following general formula: 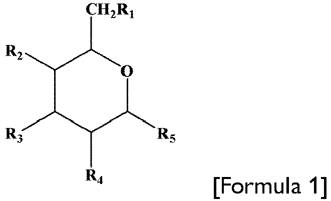 wherein
R1 to R5 are each independently selected from protected or unprotected hydroxyl group, protected or unprotected amino group, and sugar residue connected by glycosidic bond, and the sugar residue meets the requirements of Formula 1;
the polyamine compound has the following general formula: 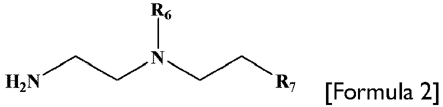 wherein
R6 is selected from hydrogen atom or 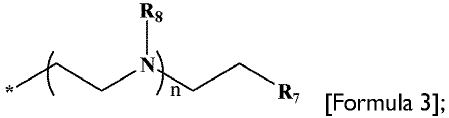
R7 is selected from protected or unprotected amino group;
R8 is selected from hydrogen atom or R6.

2. The polycationic polysaccharide according to claim 1, wherein the structural formula of the polycationic polysaccharide is one of the following structures: 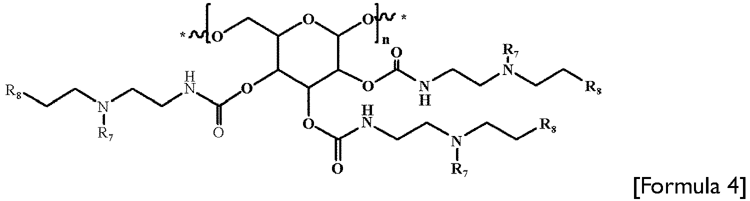 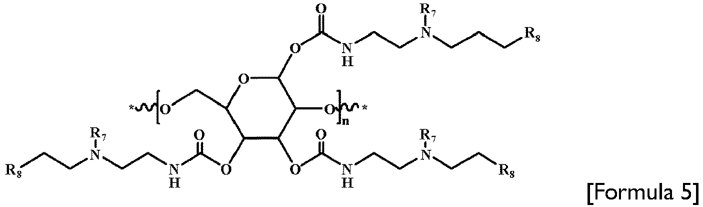 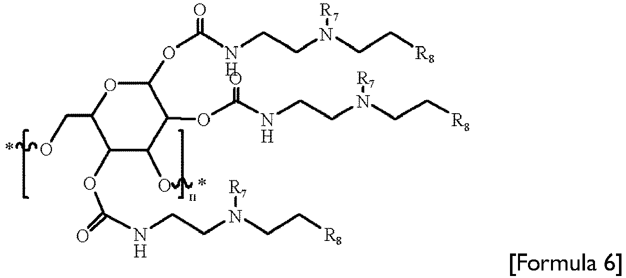 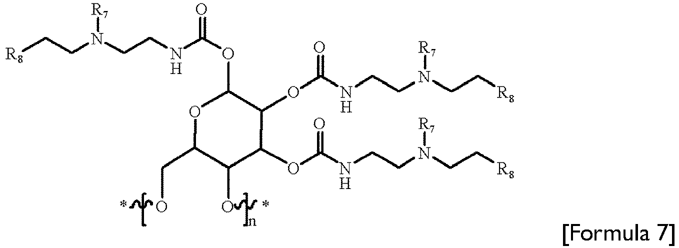
formula 4 is a polycationic polysaccharide composed of a polysaccharide molecule with (1→6) glycosidic bond as the main chain and grafted by a polyamine compound;
formula 5 is a polycationic polysaccharide composed of a polysaccharide molecule with (1→5) glycosidic bond as the main chain and grafted by a polyamine compound;
formula 6 is a polycationic polysaccharide composed of a polysaccharide molecule with (1→4) glycosidic bond as the main chain and grafted by a polyamine compound;
formula 7 is a polycationic polysaccharide composed of a polysaccharide molecule with (1→3) glycosidic bond as the main chain and grafted by a polyamine compound;
wherein
R6 is selected from hydrogen atom or 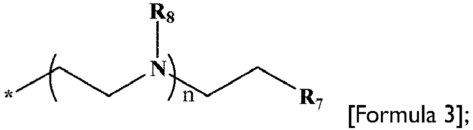
R7 is selected from protected or unprotected amino group;
R8 is selected from hydrogen atom or R6.

3. The polycationic polysaccharide according to claim 1 or 2, wherein the molecular weight of the polyamine compound is less than 500 Daltons, and the polyamine compound is any one of the following compounds:
| No | Compound | Structure |
|---|---|---|
| 1 | compound 1 | 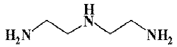 |
| 2 | compound 2 | 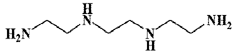 |
| 3 | compound 3 | 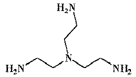 |
| 4 | compound 4 | 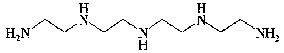 |
| 5 | compound 5 | 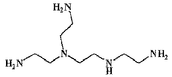 |
| 6 | compound 6 | 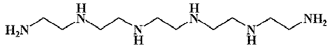 |
| 7 | compound 7 | 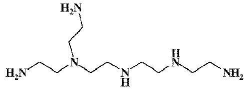 |
| 8 | compound 8 | 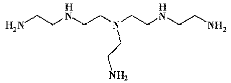 |
| 9 | compound 9 | 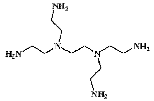 |
| 10 | compound 10 | 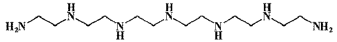 |
| 11 | compound 11 | 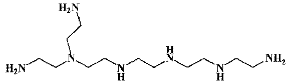 |
| 12 | compound 12 | 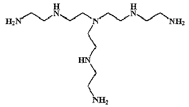 |
| 13 | compound 13 | 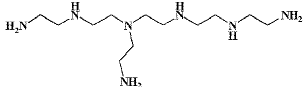 |
| 14 | compound 14 | 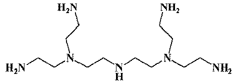 |
| 15 | compound 15 | 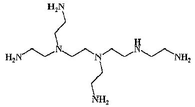 |
| 16 | compound 16 | 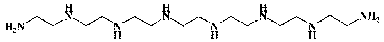 |
| 17 | compound 17 | 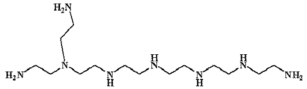 |
| 18 | compound 18 | 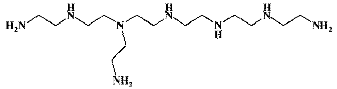 |
| 19 | compound 19 | 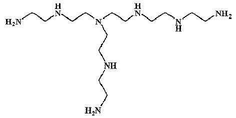 |
| 20 | compound 20 | 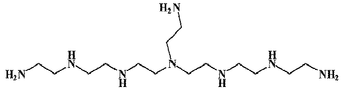 |
| 21 | compound 21 | 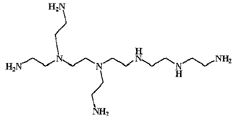 |
| 22 | compound 22 | 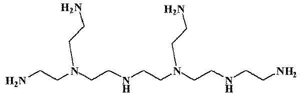 |
| 23 | compound 23 | 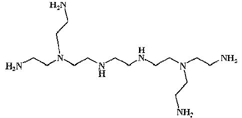 |
| 24 | compound 24 | 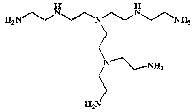 |
| 25 | compound 25 | 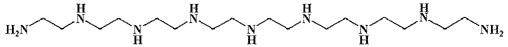 |
| 26 | compound 26 | 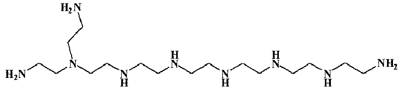 |
| 27 | compound 27 | 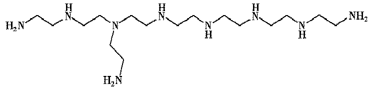 |
| 28 | compound 28 | 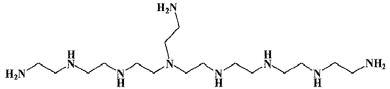 |
| 29 | compound 29 | 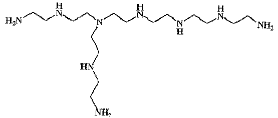 |
| 30 | compounc 30 | 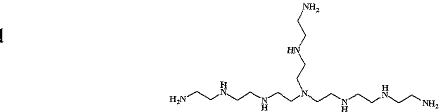 |
| 31 | compound 31 | 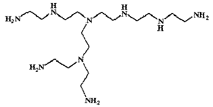 |
| 32 | compound 32 | 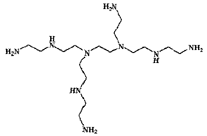 |
| 33 | compound 33 | 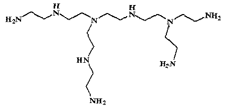 |
| 34 | compound 34 | 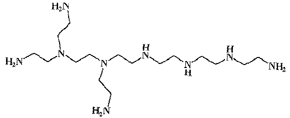 |
| 35 | compound 35 | 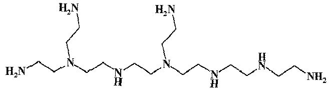 |
| 36 | compound 36 | 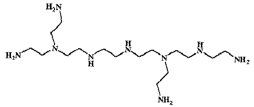 |
| 37 | compound 37 | 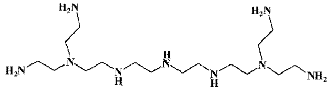 |
| 38 | compound 38 | 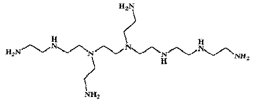 |
| 39 | compound 39 | 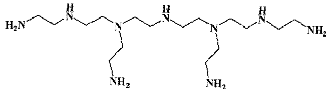 |
| 40 | compound 40 | 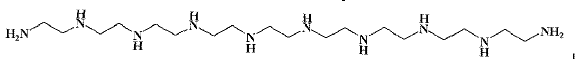 |
| 41 | compound 41 | 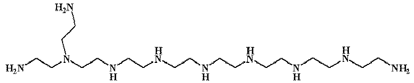 |
| 42 | compound 42 | 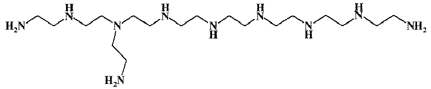 |
| 43 | compound 43 | 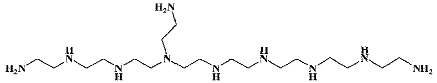 |
| 44 | compound 44 | 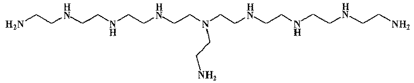 |
| 45 | compound 45 | 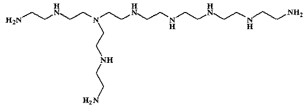 |
| 46 | compound 46 | 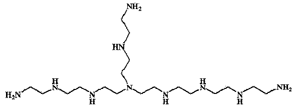 |
| 47 | compound 47 | 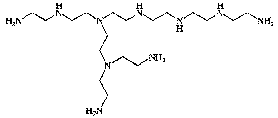 |
| 48 | compound 48 | 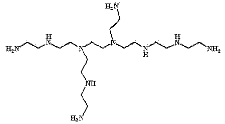 |
| 49 | compound 49 | 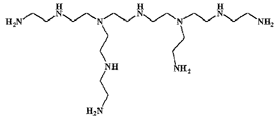 |
| 50 | compound 50 | 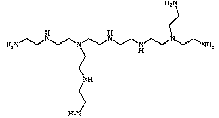 |
| 51 | compound 51 | 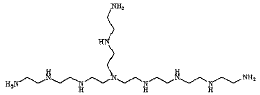 |
| 52 | compound 52 | 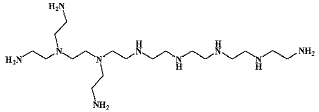 |
| 53 | compound 53 | 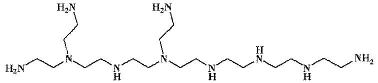 |
| 54 | compound 54 | 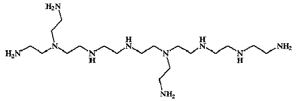 |
| 55 | compound 55 | 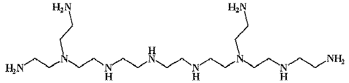 |
| 56 | compound 56 | 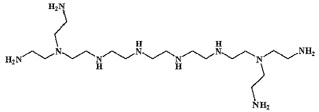 |
| 57 | compound 57 | 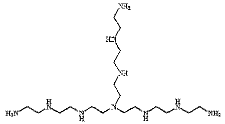 |
| 58 | compound 58 | 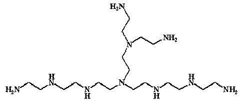 |
| 59 | compound 59 | 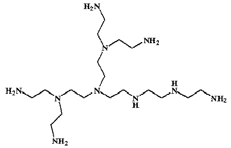 |
| 60 | compound 60 | 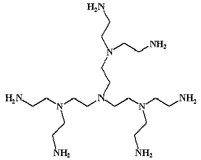 |
| 61 | compound 61 | 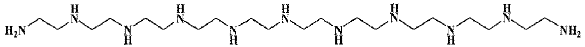 |
| 62 | compound 62 | 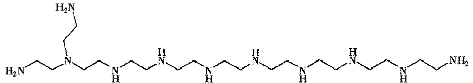 |
| 63 | compound 63 | 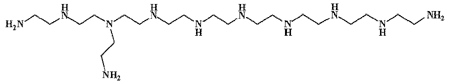 |
| 64 | compound 64 | 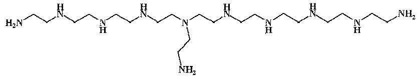 |
| 65 | compound 65 | 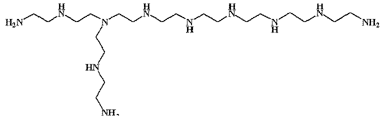 |
| 66 | compound 66 | 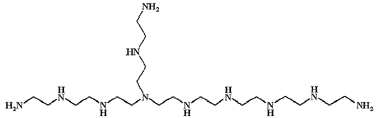 |
| 67 | compound 67 | 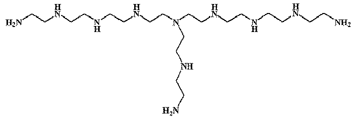 |
| 68 | compound 68 | 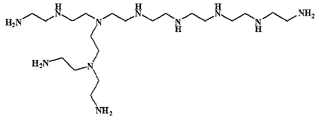 |
| 69 | compound 69 | 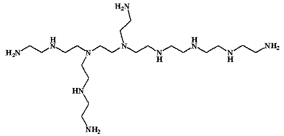 |
| 70 | compound 70 | 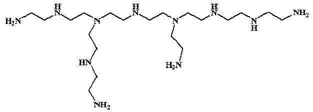 |
| 71 | compound 71 | 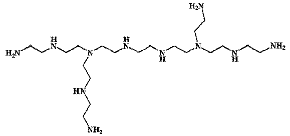 |
| 72 | compound 72 | 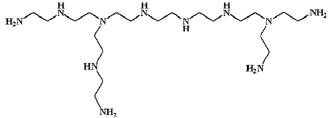 |
| 73 | compound 73 | 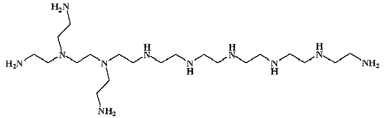 |
| 74 | compound 74 | 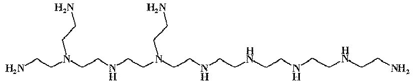 |
| 75 | compound 75 | 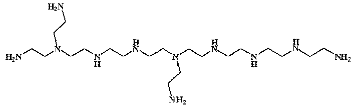 |
| 76 | compound 76 | 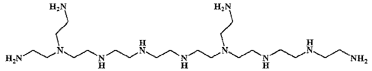 |
| 77 | compound 77 | 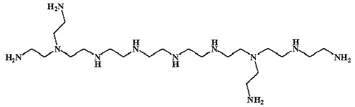 |
| 78 | compound 78 | 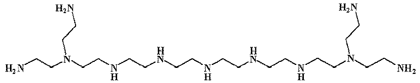 |
| 79 | compound 79 | 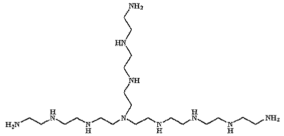 |
| 80 | compound 80 | 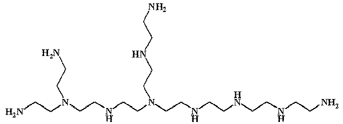 |
| 81 | compound 81 | 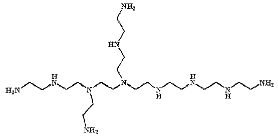 |
| 82 | compound 82 | 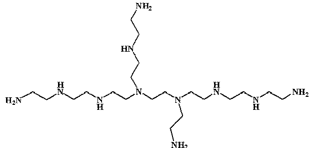 |
| 83 | compound 83 | 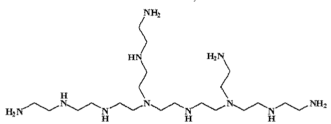 |
| 84 | compound 84 | 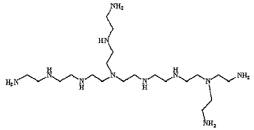 |
| 85 | compound 85 | 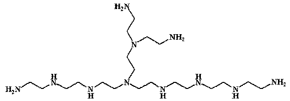 |
| 86 | compound 86 | 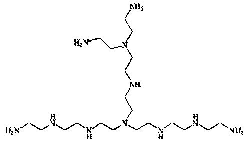 |
| 87 | compound 87 | 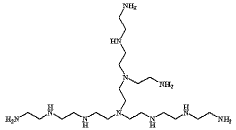 |
| 88 | compound 88 | 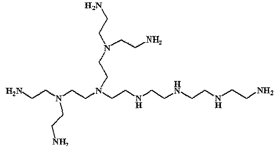 |
| 89 | compound 89 | 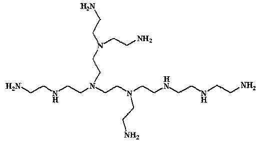 |
| 90 | compound 90 | 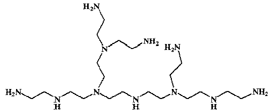 |
| 91 | compound 91 | 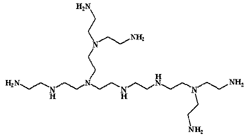 |
| 92 | compound 92 | 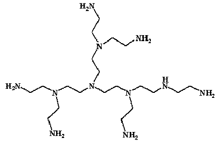 |
| 93 | compound 93 | 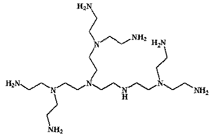 |
| 94 | compound 94 | 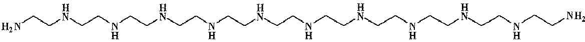 |
| 95 | compound 95 | 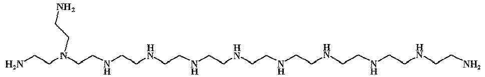 |
| 96 | compound 96 | 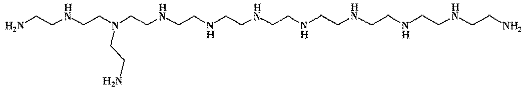 |
| 97 | compound 97 | 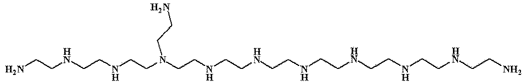 |
| 98 | compound 98 | 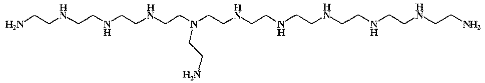 |
| 99 | compound 99 | 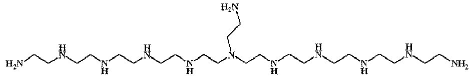 |
| 100 | compound 100 | 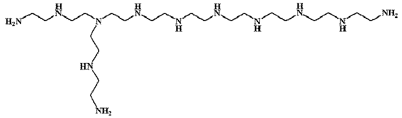 |
| 101 | compound 101 | 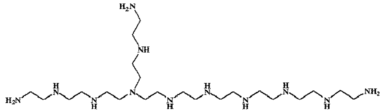 |
| 102 | compound 102 | 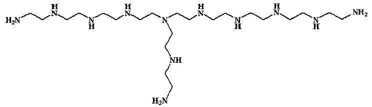 |
| 103 | compound 103 | 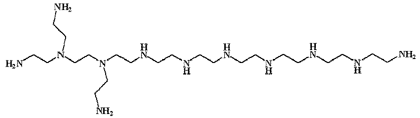 |
| 104 | compound 104 | 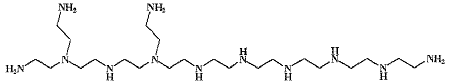 |
| 105 | compound 105 | 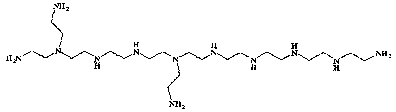 |
| 106 | compound 106 | 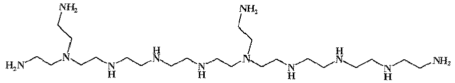 |
| 107 | compound 107 | 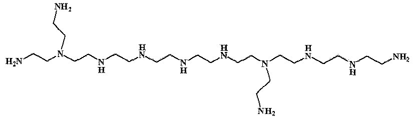 |
| 108 | compound 108 | 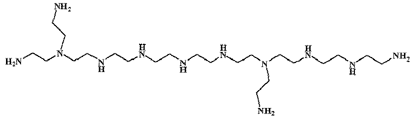 |
| 109 | compound 109 | 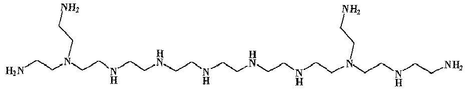 |
| 110 | compound 110 | 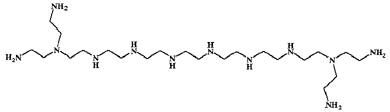 |
| 111 | compound 111 | 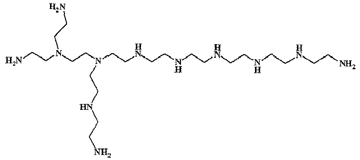 |
| 112 | compound 112 | 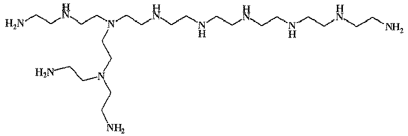 |
| 113 | compound 113 | 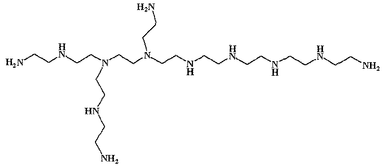 |
| 114 | compound 114 | 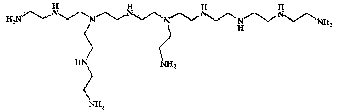 |
| 115 | compound 115 | 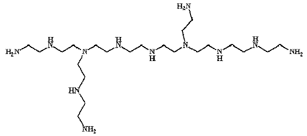 |
| 116 | compound 116 | 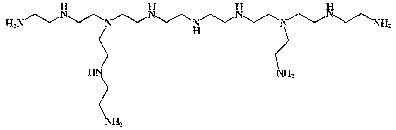 |
| 117 | compound 117 | 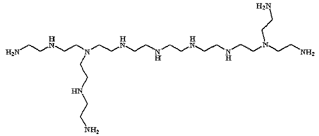 |
| 118 | compound 118 | 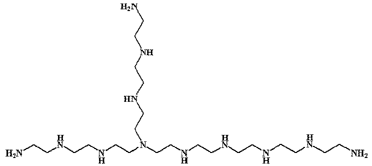 |
| 119 | compound 119 | 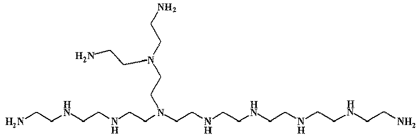 |
| 120 | compound 120 | 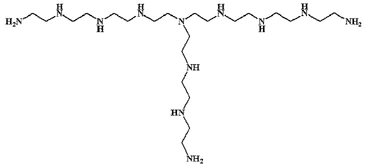 |
| 121 | compound 121 | 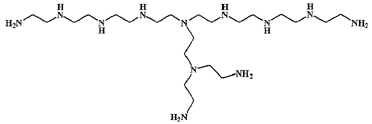 |
| 122 | compound 122 | 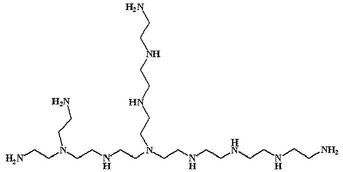 |
| 123 | compound 123 | 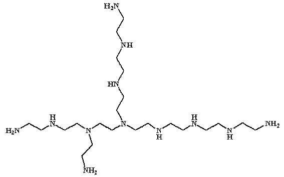 |
| 124 | compound 124 | 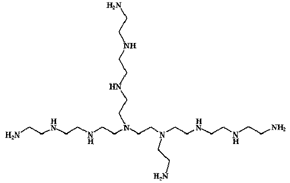 |
| 125 | compound 125 | 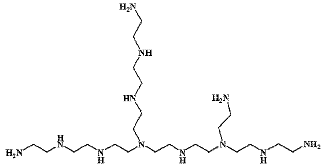 |
| 126 | compound 126 | 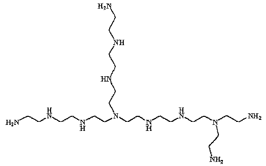 |
| 127 | compound 127 | 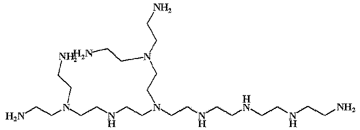 |
| 128 | compound 128 | 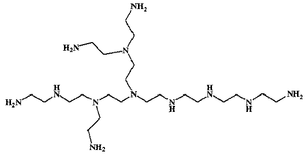 |
| 129 | compound 129 | 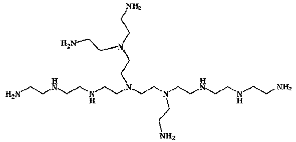 |
| 130 | compound 130 | 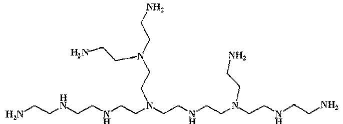 |
| 131 | compound 131 | 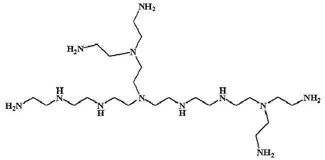 |
| 132 | compound 132 | 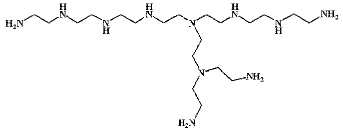 |
| 133 | compound 133 | 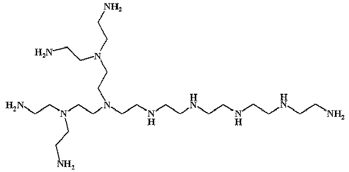 |
| 134 | compound 134 | 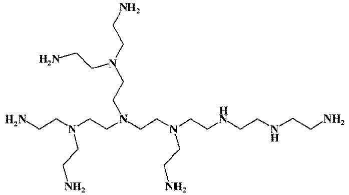 |
| 135 | compound 135 | 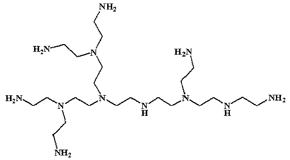 |
| 136 | compound 136 | 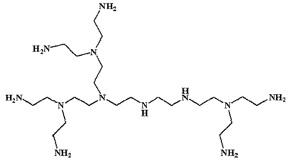 |
| 137 | compound 137 | 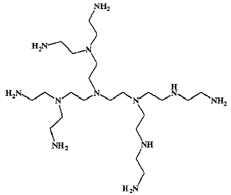 |
| 138 | compound 138 | 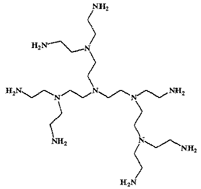 |
| 139 | compound 139 | 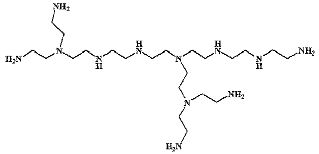 |
| 140 | compound 140 | 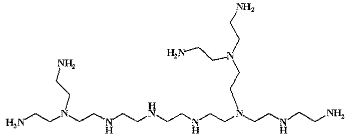 |
| 141 | compound 141 | 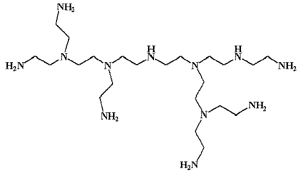 |
| 142 | compound 142 | 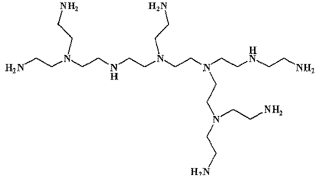 |

4. The polycationic polysaccharide according to claim 3, wherein the number of sugar units in the structure of the polysaccharide is 2 to 2000.

5. Use of the polycationic polysaccharide according to any one of claims 1 to 4 as an antibacterial material.

6. The use according to claim 5, wherein the antibacterial material achieves the effect of killing bacteria by destroying the biofilm structures of the bacteria.

7. The use according to claim 6, wherein the antibacterial material is used for the preparation of a medicament or a medical device for the prevention or treatment of Gram-negative and/or Gram-positive bacterial infection.

8. The use according to claim 6, wherein the antibacterial material is used as a biomedical functional material or a biomedical device.

9. The use according to claim 5, wherein the antibacterial material is an antibacterial functional material, including a daily chemical product, a packaging product, and a home improvement product with antibacterial functions.

10. An antibacterial agent, prepared from the polycationic polysaccharide according to any one of claims 1-4 as an active ingredient and a pharmaceutically acceptable adjuvant.

11. An antibacterial medical device, prepared from the polycationic polysaccharide according to any one of claims 1-4 as an active ingredient and a pharmaceutically acceptable adjuvant.

12. Use of the polycationic polysaccharide according to any one of claims 1 to 4 as a medicament for the treatment of chronic inflammatory disease.

13. The use according to claim 13, wherein the medicament for the treatment of chronic inflammatory disease includes the medicament for the prevention of surgical wound infection, and the medicament for the prevention of scalding wound infection.
